# EUROPEAN PATENT SPECIFICATION

(11) **EP 1 771 121 B1**
(45) Date of publication and mention of the grant of the patent: **22.06.2011**
(21) Application number: 05758054.0
(22) Date of filing: 29.06.2005
(51) Int. Cl.: A61B 18/20

(54) **MANIPULATION OF HAIR GROWTH**
HAARWUCHSMANIPULATION
MANIPULATION DE LA POUSSE DES POILS

(30) Priority: 09.07.2004 US 586686 P
(43) Date of publication of application: 11.04.2007
(73) Proprietor: Braun GmbH, 61476 Kronberg (DE)
(72) Inventor: BEERWERTH, Frank, 65558 Kaltenholzhausen (DE); HARTTMANN, Brigitte, 65527 Niedernhausen (DE); NAHLEN, Patric, 61352 Bad Homburg (DE)
(86) International application number: PCT/EP2005/006973
(87) International publication number: WO 2006/005443

(56) References cited:
- EP-A- 0 724 894
- EP-A- 0 913 127
- EP-A- 1 358 907
- WO-A-95/03089
- WO-A-98/51235
- WO-A-99/34867
- WO-A-02/094116
- WO-A-2004/073537

## Description

### TECHNICAL FIELD

This invention relates to hair removal.

### BACKGROUND

Many procedures are used to remove unwanted hair including shaving, electrolysis, plucking, laser and light therapies and injection of therapeutic antiandrogens. Laser and other optical radiation sources have been utilized in devices for performing a variety of dermatology and other medical/cosmetic procedures including as preventing or reducing hair growth. By using radiation of light in a specific wavelength range, with an exactly tuned intensity and pulse sequence, it is possible to selectively heat the wells of a root of a hair stronger than the ambient skin and to thereby damage them, while the ambient dermal tissue remains almost intact. Such procedures typically target a chromophore in the tissue of the individual being treated, which, depending on the procedure may be melanin, hemoglobin, lipid, water, pigment of a tattoo etc.

EP 0 913 127 A2 discloses a device for eliminating hairs and/or atrophying hair follicles, the device comprising a housing having an opening to allow radiation to pass therethrough, a treatment light source disposed within the housing, a sensor light source disposed within the housing, which is designed to light the epidermis through the opening, and an optical sensor consisting of a micro-telecamera, which is designed to retrieve through the opening the images of the portion of the epidermis reached by the treatment light source.

### SUMMARY

In general, devices for the manipulation of hair growth, for example, the reduction or prevention of hair growth are described.

One embodiment includes a device for reducing hair growth as defined in claim 1.

In some instances, the light source is a light emitting diode.

In some instances, the device includes a handle disposed on an outer surface of the housing.

In some instances, the device is configured to be held in the hand of a user.

In some instances, the device also includes a sensor responsive to motion, wherein the sensor is conductively linked to the light source. The sensor can be, for example, a mechanical sensor or an optical sensor. The sensor can be configured, for example, to signal the light source to emit divergent light upon detection of relative movement between the device and the skin of the subject. Alternatively, the sensor can be configured to prevent the light source from emitting divergent light when no relative motion is detected between the device and the skin of the subject.

In some instances, the device includes a plurality of light emitting diodes.

In some instances, the device includes an array of light emitting diodes.

In some instances, the device includes a plurality of arrays of light emitting diodes.

In some instances, the device includes the light emitting diodes are illuminated in a mutually pulsed manner. The light emitting diodes can also be illuminated in a continuous manner. In some embodiments the light emitting diodes are illuminated at a single wavelength. In other embodiments, the light emitting diodes are illuminated at a plurality of wavelengths.

In some instances, the power source is disposed within the housing. The power source can be, for example, a battery. In other instances, the power source is disposed outside of the housing.

A method for reducing hair growth of a subject is also described. The method includes providing a device comprising a divergent light source; and illuminating a portion of skin of a subject comprising hair with a divergent light source at a wavelength and for a time sufficient to reduce hair growth.

In some instances, the divergent light source is a light emitting diode.

In some instances, the divergent light source emits light having a wavelength between about 700 nanometers and about 1100 nanometers.

In some instances, the light efficiency of the divergent light source is at least about 0.1 Watts.

In some instances, the light efficiency of the divergent light source is between about 1 Watts and about 3 Watts.

In some instances, the divergent light source has an energy density of between about 2 J/cm² and about 50 J/cm².

In some instances, the divergent light source is illuminated in a pulsed manner.

In some instances, the length of a pulse is between about 1 nanoseconds and about 100 milliseconds.

In some instances, the divergent light source is illuminated in a continuous manner.

In some instances, the divergent light source is illuminated for between about 1 nanoseconds and about 5 seconds.

In some instances, the skin is illuminated with a plurality of light emitting diodes.

In some instances, the skin is illuminated with an array of light emitting diodes.

In some instances, the skin is illuminated with a plurality of arrays of light emitting diodes.

In some instances, the light emitting diodes are illuminated in a mutually pulsed manner.

In some instances, the light emitting diodes is illuminated in a continuous manner.

In some instances, the light emitting diodes are illuminated at a single wavelength.

In some instances, the light emitting diodes are illuminated at a plurality of wavelengths.

In some instances, the skin is illuminated for at a wavelength and for a time sufficient to eliminate hair growth.

In another embodiment, a device for reducing hair .growth on a subject is described. The device includes a housing comprising an opening to allow radiation to pass therethmugh; a treatment light source disposed within the housing; a sensor light source disposed within the housing; and a detector for detecting the presence of skin directly in front of the opening conductively linked to the sensor light source and the treatment light source, wherein the treatment light source and the sensor light source are configured to emit substantially collinearly from the opening of the housing.

In another embodiment, a device for reducing hair growth on a subject is described. The device includes a housing comprising an opening to allow radiation to pass therethrough; a treatment light source disposed within the housing; a sensor light source disposed within the housing; and a detector for detecting the presence of skin directly in front of the opening conductively linked to the sensor light source and the treatment light source, wherein the treatment light source and the sensor light source share at least a portion of an optical path disposed within the housing.

In some instances, the treatment light source is a light emitting diode.

In some instances, the treatment light source is a laser.

In some instances, the treatment light source is a glow discharge lamp.

In some instances, the discharge lamp is a pulsed glow lamp.

In some instances, the discharge lamp is a continuous glow lamp.

In some instances, the device is a hair removal device.

In some instances, the detector is configured to detect light scattered from skin of the user.

In some instances, the device also includes a controller operably linked to the detector and the treatment light source.

In some instances, the controller is configured to switch on the treatment light source upon detection of scattered light from the skin of the user.

In some instances, the sensor light source emits light in the range of visible wavelengths.

In some instances, the treatment light emits light ranging from about 600 nm to about 1100 nm.

In some instances, the device also includes a motion sensor conductively linked to the detector.

In some instances, the motion sensor is disposed within the housing.

In some instances, the motion sensor is disposed on an outer surface of the housing.

In some instances, the motion sensor is a mechanical sensor.

In some instances, the motion sensor is an optical sensor.

Another method of reducing the hair growth of a subject is also described. The method includes; providing a device, the device comprising a housing comprising an opening to allow radiation to pass therethrough; a treatment light source disposed within the housing; a sensor light source disposed within the housing ; and a detector for detecting the presence of skin directly in front of the opening conductively linked to the sensor light source and the treatment light source, wherein the treatment light source and the sensor light source are configured to emit from the opening of the housing substantially collinearly; exposing a portion of skin of the user to the sensor light source from the device, and exposing a portion of skin of the user to the treatment light source from the device at an energy and for a time sufficient to reduce the hair growth of the subject.

Another method of reducing the hair growth of a subject is also described. The method includes providing a device, the device comprising a housing comprising an opening to allow radiation to pass therethrough; a treatment light source disposed within the housing; a sensor light source disposed within the housing; and a detector for detecting the presence of skin directly in front of the opening conductively linked to the sensor light source and the treatment light source, wherein the treatment light source and the sensor light source share at least a portion of an optical path disposed within the housing; exposing a portion of skin of the user to the sensor light source from the device, and exposing a portion of skin of the user to the treatment light source from the device at an energy and for a time sufficient to reduce the hair .growth of the subject.

In some instances, the light source has an energy density from between about 2 J/cm2 and about 50 J/cm2.

In some instances, the skin is exposed to the light source for between about 1 nanosecond and about 5 seconds.

In some instances, the light source is pulsed.

Another method of reducing hair growth of a subject is also described. The method includes providing a device comprising a divergent light source; and exposing a portion of the skin of a user to a light source having an energy density between about 2 J/cm² and about 15 J/cm² for a time sufficient to reduce hair growth.

Another method of reducing hair growth of a subject is also described. The method includes providing a device comprising a divergent light source; and exposing a portion of the skin of a user to a light source having an energy density between about 2 J/cm² and about 25 J/cm² and a light efficiency between about 1 W and about 5 W for a time sufficient to reduce hair growth.

In some instances, the energy density is between about 5 J/cm² and about 10 J/cm².

In some instances, the light source has a light efficiency between about 1 W and about 5 W.

In some instances, the light source is emitted for between about 10 ms and about 40 ms.

In some instances, the light source is pulsed.

In some instances, the length of a pulse is between about 10 ms and about 40 ms.

In some instances, the light produces a radiant area in the range between about 0.3 mm² and about 1 mm² at between 15 J/cm²/5W/10ms and 2 J/cm²/1W/20ms.

In some instances, the light source is a laser.

In some instances, the light source is a light emitting diode.

In some instances, the light source is a glow discharge lamp.

In another embodiment, a hair removal device is described. The hair removal device includes a housing, comprising an opening from which to emit light; and a light source disposed within the housing, wherein the light source emits light having an energy density between about 2 J/cm² and about 15 J/cm².

In another embodiment, a hair removal device is described. The hair removal device includes a housing, comprising an opening from which to emit light; and a light source having an energy density between about 2 J/cm² and about 25 J/cm² and a light efficiency between about 1 W and about 5 W for a time sufficient to reduce hair growth.

In some instances, the light source is a laser.

In some instances, the light source is a light emitting diode.

In some instances, the light source is a glow discharge lamp.

In some instances, the device includes a plurality of light sources.

In some instances, the light source is configured to emit light in a pulsing manner.

In some instances, the light source is configured to emit light in a continuous manner.

In some instances, the devices provide improved safety relative to some commercially available methods and devices as the devices can include a safety feature that prevents damaging light from being applied to unwanted regions, for example, an eye. Moreover, embodiments including an LED light source can have substantially lowered safety requirements relative to the requirements of laser light sources and also lower costs relative to laser light instances, the device can be a compact, hand held device, which can be used by a person in the home without medical training. Thus, in some instances the devices and methods do not require application in a professional setting.

In some embodiments the device includes a safety system that works independently of the dimensions of the treatment area. The device can include a sensor beam, which can be part of a safety system, where the sensor beam uses a portion of the optical path of the treatment beam, thus allowing for a compact configuration of the device. The more compact design can allow the device to more easily be correctly positioned on the skin and more easily handled by the user. Is some instances the compact design also allows reduced production costs. In still other embodiments, this configuration also allows for an error in the beam deflection to be automatically detected by the safety system.

In some embodiments, the devices can be used without the application of a coolant to the skin of a subj ect, which can provide for easier manipulation of the device. Moreover, in a relatively small light beam is used, which can increase safety and reduce thermal damaging of the skin.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG 1 is a cross sectional view of a hair removal device.
FIG 2 is depicts an array of light emitting diodes.
FIG 3 is a cross-sectional view of a hair removal device featuring a sensor light source, a treatment light source, and a detector for detecting reflected light.
FIG 4a is a cross-sectional view of the hair removal device of FIG 3, showing the optical path of only the sensor light source. ,
FIG 4b is a cross-sectional view of the hair removal device of FIG 3, showing the optical path of only the treatment light source.
FIG.4c is a cross-sectional view of the hair removal device of FIG 3, showing the optical path of the reflected light and the detector for detecting that light.

Like reference symbols in the various drawings indicate like elements.

### DETAILED DESCRIPTION

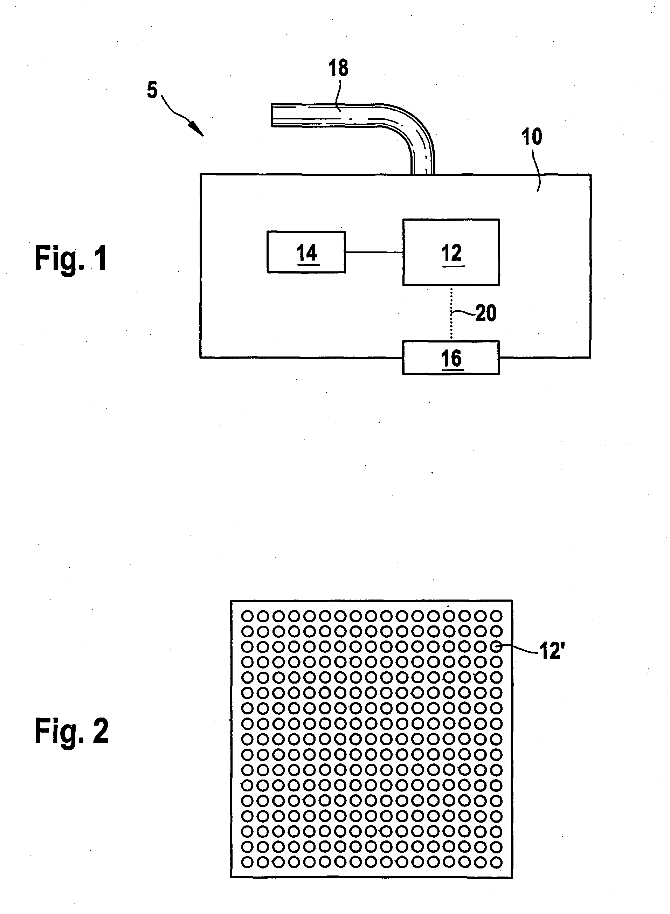

A hair removal device 5 is depicted in Fig. 1. The device includes a housing 10, a light source 12, a power supply 14, an aperture 16, and a handle 18.

### LEDs for Manipulation of Hair Growth

Referring to Fig. 1, the hair removal device 5 includes a light source 12 disposed within the housing. The light source is a divergent light source, for example an LED. The device also includes and a power supply 14, which is electrically connected to the light source 12. An aperture 16 is positioned within the housing, optically linked to the light source, to allow the light 20 form the light source 12 to reach the skin of the user.

The light source is preferably a divergent light source, such as a light emitting diode (LED). In some embodiments the light source is a plurality of LED's, for example an array of LEDs as depicted in Fig. 2 or a plurality of arrays of LED's. The plurality of LEDs can emit light in the infrared wavelength range, for example, in the range between 700nm and 1100nm. In general, the LEDs such as those depicted in Fig. 2 produce light having a high intensity.

The divergent light source can be, for example, equipped with light emitting diodes of different wavelengths which are suited to generate the high luminous density required for the hair removal. By combining the different wavelengths appropriately, the light system can be adapted in an optimum manner to the property of the skin to be treated and the hairs to be removed.

For the treatment of larger skin sections, several LEDs 12 or arrays of LEDs 12' can be located in the device 5. The LEDs or arrays of LEDs can be controlled in a simultaneous or in a mutually pulsed or in a continuous fashion. The arrangement of the LEDs can be further selected such that the complete surface of the skin can be irradiated via movement of the device over the skin.

The light efficiency of the light source 12 can be, for example, greater than about 0.1 W (e.g., greater than about 0.3, greater than about 0.5, greater than about 1.0, greater than about 5.0, greater than about 7.0, etc.).

In some instances the housing 10 is configured to be held in the hand of the user. The user can apply the light to the skin by moving the device 5 over the skin sections to be treated.

Alternatively, a light source can be arranged in a base unit, with the light being guided to a hand held piece by means of light conductors.

In some instances, the device can include a sensor (not shown), for example like the sensor used in a "PC mouse", that detects the movement of the device relative to the surface (skin) to be treated. Like the PC-mouse, the sensor can either be an optical or a mechanical sensor. The sensor can be positioned, for example, within the housing 10 or alternatively can be positioned on an outer surface of the housing 10. In preferred embodiments, the sensor is operably linked to the light source 12. The sensor can in some instances simplify the handling of the device, allowing it to be more easily used by a non-skilled person.

In some instances, the sensor can be connected to a controller that evaluates the movement of the device For example, if no motion is detected by the sensor, then the controller will prevent the light source from emitting light. On the other hand, when the motion detector senses motion, the controller can signal to the light source to emit light from the device. Accordingly, in order to prevent possibly injury or damage to the skin through over exposure, light emission can be interrupted if the hand piece simply rests on the skin of the user without moving.

The movement sensor can also provide information to the controller that will allow the device to act an "intelligent" fashion, for example changing the light energy, exposure time, etc. based on the information obtained from the sensor upon analysis of the skin surface. In some embodiments, the light sources are so controlled that all spots of the surface are irradiated with an exactly defined light dose, almost independent of the movement of the hand piece.

For example, the device can include a sensor, preferably an optical sensor, for the detection of the tanning degree of the skin or the recognition of the skin type. The information can be displayed at the device or can simultaneously also be used for the control and automatic adaptation of the emitted light efficiency and/or spectral composition in the infrared range.

In some embodiments, the device can include light sources, such as LEDs, emitting light in having varying wavelength. For example the device can include LEDs emitting light both the visible and the infrared wavelengths. The light source emitting visible light can be used either independently or in conjunction with a second light source, for example an infrared emitting LED. For example, the visible light emitting source can be used to indicate to the user the operational state of the lighting apparatus or alternatively serve as a warning signal of a misuse for avoiding hazards by the intense infrared radiation. For example, a Visible LED can be configured to emit a lighting sequence wherein a visible light flash is emitted shortly before the infrared LED. This can alert the user of the upcoming radiation and, for example, as a safety feature, a lid-closing action can be activated so that, in the event of a mispositioning, an injury of the eye can be prevented or reduced.

In other instances a combination of LEDs that emit light of different wavelengths can allow an adaptation of the spectral light composition, which can optimize the effects of the hair removal device on varying skin types, for example thin or sensitive portions of skin and thicker, less sensitive portions of skin.

Examples of LEDs include infrared emitting LEDs such as epoxy lens type LEDs, including LED870-66-60, which is available from Roithner Lasertechnic Vienna, Austria.

### Safety Feature for Hair Removal Devices

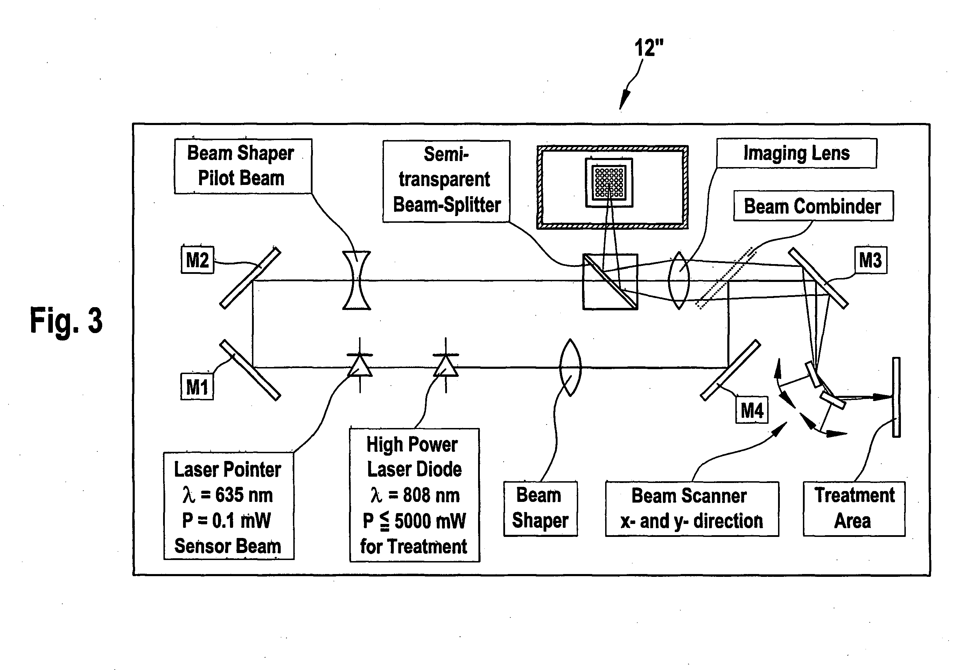

Fig. 3 depicts a schematic diagram for a light source 12" including an optical assembly that combines three different optical paths: the sensor laser beam, the treatment laser beam, and the optical imaging path for analysing the object to be treated. Some of the optical elements are used for different tasks simultaneously.

A portion of the optical path is shared for all beams, including beams leading to the scanner unit. The scanner unit includes two motor driven metallized glass mirrors to scan in x- and y-direction. Due to the fact that the scanner unit is jointly used, the sensor beam can be used as the light source for the safety detection unit.

Although the light source of 12" includes a laser, other light sources can also be used, for example an LED or a pulsed or continually working glow discharge lamp. The light source 12" can be used, for example, in a device for reducing hair growth or removing unwanted hair. In some embodiments, the device can be used by an unskilled user, for example, in the home of the user.

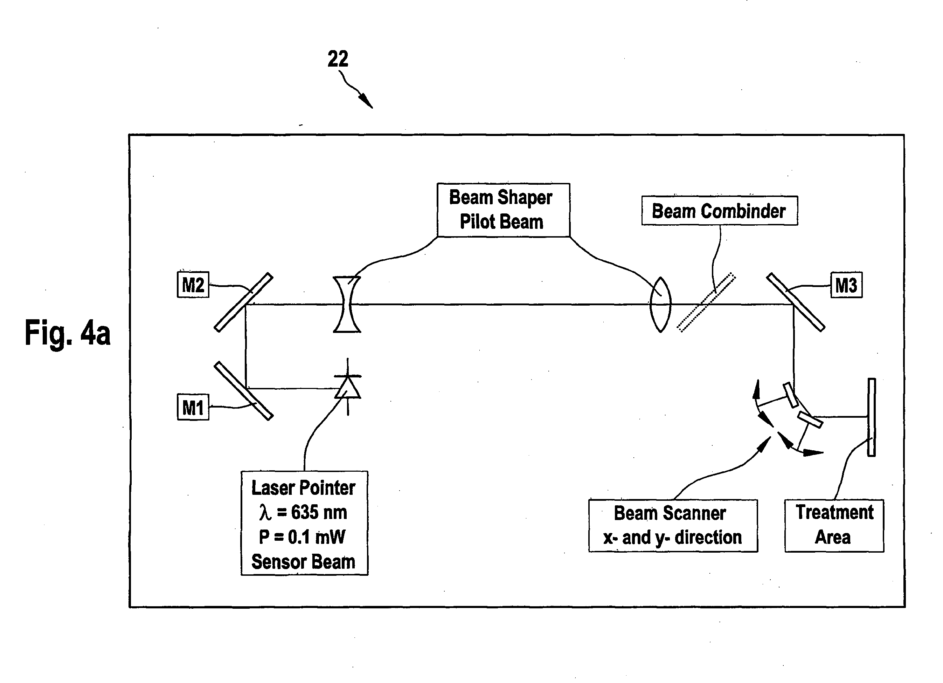

Fig. 4a depicts the optical path 22 for the sensor light source, which can be used, for example, as a safety feature. An eye can be significantly injured by high intensity light produced by means such as lasers or pulsed glow discharge lamps typically used in the treatment light source. Accordingly, a safety feature that prevents an unintended emission of light in case of improper use can, in some instances, prevent or reduce injury to an eye.

The sensor laser beam can indicate the position of the treatment laser for safety reasons. The wavelength of the sensor laser can be, for example, in the visible range up to about 650nm. In some preferred embodiments, the wavelength is 635nm of a laser diode between about 0.1mW and about 1.0 mW, for example 0.1 mW or 1.0 mW.

The output of the sensor laser diode is internally collimated. The beam diameter can be, for example between about 2 mm and about 8 mm, (e.g., between about 3 mm and about 7 mm, or about 5mm). Two metal mirrors (M1 and M2) located directly behind the laser output are provided to allow adjustment of the beam direction. Two lenses, a biconcave lens and a biconvex lens, are used to focus the pilot beam on the surface of the treatment area. This spot is the center of the treated area. The beam combinder has low reflection for wavelength below about 650nm, e.g., about 600 nm, about 550 nm, about 500 nm, etc. Accordingly, the sensor beam is transmitted with relatively low intensity loss.

The metal mirror M3 is used to adjust the combined laser beam to the center of the scanning mirrors.

The distance between both scanning mirrors, in preferred embodiment, is as low as possible, for example between about 5 mm and about 15 mm, between about 7 mm and about 13 mm, or about 10mm. Several variables can be used to determine the distance between the treatment area and the second scanning mirror including, for example, the desired scanning area, the diameter of the beam at the mirror position, the required resolution of the beam placement, and the opening of the optical path for measuring the scattered light from the skin. The distances in some embodiments are set between 50mm and 100mm.

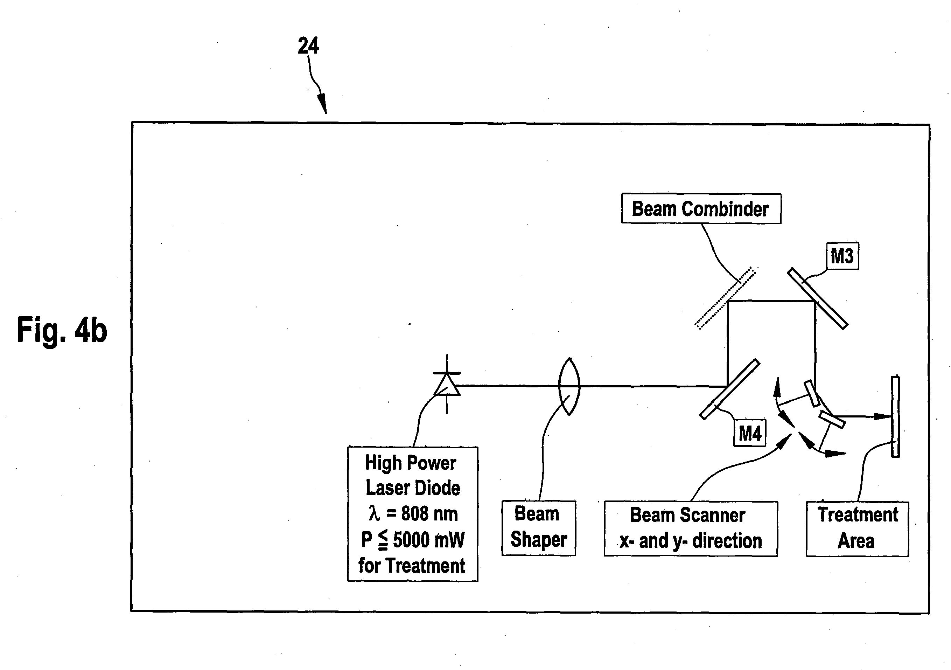

The source for the treatment radiation 24 depicted in Fig. 4b is, for example, a high power laser diode with an integrated fast axis-collimating lens. The radiation has a medium wavelength, for example, of between about 600 nm and about 1000 nm, for example between about 700 nm and about 900 nm, between about 800 nm and about 820 nm, or about 808 nm. In general, the treatment radiation source has a maximum power of up to about 5W, for example up to about 4W, about 3W, about 2W, or about 1W. The opening angle of the light beam is in vertical direction between about 0.1° and about 5°, for example between about 0.3° and about 3°, between about 0.7° and about 2°, between about 0.8° and about 1.2°, or about 1°. The opening angle of the light beam is in horizontal direction between about 4° and about 12°, for example between about 5° and about 11°, between about 6° and about 10°, between about 7° and about 9°, or about 8°. The virtual beam output area of the laser diode behind the FAC is about 200µm by about 100µm, for example between about 100 µm to about 300 µm by about 50 µm to about 150 µm. In general, it is preferred that the ratio of output area of the laser diode is between about 3/1 to about 1/1.

The beam-shaping lens is selected and placed to create an image of the beam output area of the laser diode at the treatment area. The focal length and the position of the beam shaper control the size of the image at the treatment area. The criterion for the selection is to achieved fluence between about 5J/cm² and about 20J/cm², for example between about 8 J/.cm² and about 15 J/cm², or between about 10 J/cm² and about 12 J/cm².

The mirror M4 and the beam combinder, which is a dielectric mirror with high reflection at 45° for 808nm, is used to align the treatment beam with the sensor beam. Behind the beam combinder both beams use the same path. While the reflection depicted is at 45° other reflection angles can also be used, for example angles between about 30° and about 60°, between about 35° and about 55°, or between about 40° and 50°.

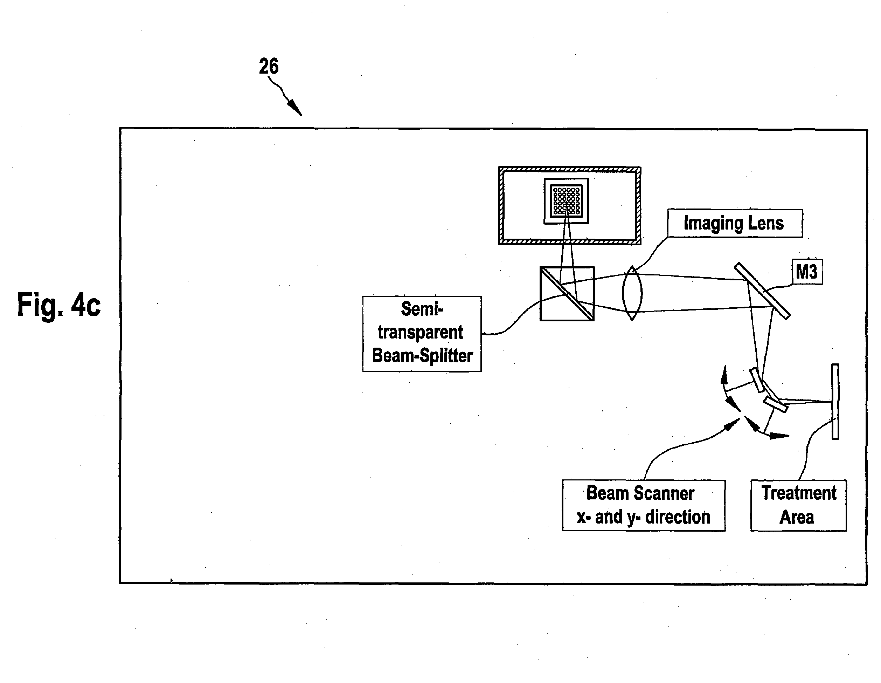

The sensor beam can be used to detect the presence of skin at the treatment position. A controller can be configured to activate the treatment beam only when skin is determined to be in the treatment area. In general, the sensor beam is configured such that it can detect the presence of skin in front of the opening of the device without being positioned to be in contact directly with the skin. As depicted in Fig. 4c, the splitting of the beam reflected from or broken by the skin from the sensor laser beam is performed by means of partially transparent or polarized optical elements 26.

The device can also include a sensor system can for detecting the relative movement of the device between the skin to assure that the complete area is irradiated during the treatment. The treatment plane can be pictured on a photo diode by means of an imaging lens in the pilot laser beam path, said photo diode having at least two sensitive areas (one in the center and the second configured as a ring).

As can be seen in Fig. 3, the optical beam path is identical for the sensor laser and the treatment laser from the Beam-Combinder through the aperture, such that the sensor laser and the treatment laser emit collinearly.

In some instances, the device can include a safety system including a combination of two detector systems, the contact and movement sensor and the optical sensor to detect the reflection and scattering properties of the object at the treatment location. The safety system can be configured irrespective of whether the treatment laser is in use or in stand-by position, so that an examination before or during the treatment can be performed.

In some instances, the contact and movement detection can be a standard mechanical and/or electrical component used in mouse devices, for example optical or mechanical mouses. This component can be located in the device and can be positioned adjacent to the area to be treated. In the instance an optical motion sensor is used, an LED illuminates a small area of the skin. A small imaging sensor with low resolution then receives the reduced image of this area, and the movement of the hand piece over the skin is calculated from the consecutive analysis of this image.

This motion sensor system can recognize whether any kind of material is in contact with the hand piece and can also generate movement data, which can additionally be used as a control of the hair removal device. The detection of relative motion between the device and the skin of the user can be used to aid in the treatment of the whole skin surface regardless of how the device is moved.

The sensor for skin recognition is based on the optical properties of the skin, the reflection and the scattering. The sensor light source used to detect the presence of skin has a wavelength of between about 400 nm and about 800 nm, for example between about 500 nm and about 700 nm, or about 600nm, which is nearly transparent.

The skin surface provides for the wave length to be applied an average, not exactly defined diffuse reflection, there is almost no directional reflection (mirror effect). With the applied wavelength, the skin itself provides no absorption. Only melanin parts in case of tanned skin and in the hair do absorb the light used for the testing. The skin provides a high scattering of the light, which causes the production of a so-called "halation" around the area where the light penetrates the skin. The detection of this halation is an essential element of the skin recognition. The light is scattered inside the skin at each cell membrane due to variations of the index of refraction. This kind of halation does not exist, when light is reflected by opaque material. Accordingly, the difference in the reflected beam is detected in the optical sensor disposed within the housing of the hair removal device.

The scattered reflection from an eye is similar to the scattering of pure skin. To differentiate between eye and skin the directly reflected beam is analysed. The beam intensity, mirrored from the surface of the eye is generally more than one order of magnitude higher than the intensity of the diffuse reflected light off the surface of the skin. The surface of the cornea of the eye has a high directional reflection, almost no diffuse reflection and a low internal scattering. These properties are recognized by means of the detected back-scattering of the eye.

To detect the presence of skin in front of the device opening the quotient of the scattered and reflected light can be calculated. If the quotient of scattered to reflected light is higher than a defined threshold, it can be determined that there is skin in the treatment area.

A second threshold therefore can further be defined for the measured intensity at the center diode. If it is determined that this intensity is above a defined threshold, it is determined that the sensor laser hits an eye. In instances where intensity found to be above this threshold, the treatment laser treatment will not be started, or in the event the treatment laser was already emitting light, the treatment laser will be interrupted, thus cancelling the emission of light.

The treatment area is exposed through an opening in the housing and can be defined by the surface of a PIN diode array or a dual PIN diode with a sensitive center area and a second circular sensitive area. Because both the sensor laser beam and the imaging optic use the same scanner mirrors, the image on the detector doesn't move while the sensor laser beam is scanned over the treatment area.

The intensity of the diffuse reflected light is measured with the center PIN diode: The circular part of the detector senses the intensity of the scattered light. A PIN diode is a semiconductor device that can operate as a variable resistor, for example, at RF and microwave frequencies.

Generally, where the device is used for reducing hair growth of a subject, the sensor light source emits a light used to analyse the object in the treatment area prior to emission of light from the treatment light source. In case the hair removal device is moved relative to the object in the treatment area, the treatment is interrupted and the new treatment area is scanned for analysis. Once it is determined that it is safe to proceed, the treatment is continued.

The power requirement of the device is strongly related to the power of the laser source. In total the required electrical power is generally between about 2 and 4 times the light output power, for example about three times the light output power. Thus, in general, the electrical power is in the range of 10W to 25W.

In some instances, the power requirements are greater than can be suitably-supplied using a battery. Accordingly, an alternative power supply such as a power plug is used.

In some embodiments the device is configured to be held in the hand of the user. Such a device may be, for example a cube having sides between about 5 cm to about 15 cm, for example between about 7 cm and about 13 cm, between about 9 cm and about 11 cm, or about 10cm. In some embodiments, the device has a handle disposed on an outer surface of the housing.

### Hair Removal Devices Using Radiant Sources Which Do Not Require Use of a Coolant

Hair removal by means of light energy is generally based on a damaging of the hair follicle by selective photo thermolysis. If skin is irradiated with light, for example light within wavelength range of about 600 nm to about 1100 nm, (e.g., 700 nm to about 1000 nm, 800 to about 900 nm), the energy of this light is selectively absorbed by melanin. Melanin is a colorimeter, which exists in the dermis only in the hair stem and in the hair follicle and which determines the hair colour. In addition, melanin particles exist in the epidermis and its number depends on the colour of the skin and its tanning degree.

Thus, irradiation of skin as described above leads in the dermis to a selective heating of hair stem, hair follicle and direct environment. In case of a sufficient power supply these areas are heated to such an extent that the hair-producing cells are damaged. The treated hair is ejected and the further hair growth is slowed down or stopped. As haemoglobin in the blood and tissue water absorb light in this wavelength range only very poorly, only a scattering and reflection occur in the range of the ambient structures of the skin, but no heating through absorption.

With the hair removal by means of light energy, the pulse lengths are so selected that they are below the thermal relaxation time of the hair follicle of between about 40 nm and about 100 nm. The energy supplied during a pulse should be large enough to cause thermal damaging of the hair follicle and hair bulbus.

The distinctly smaller melanin particles in the epidermis absorb less energy than the larger hair follicles. In addition, they have a larger surface volume ratio and emit the supplied heat distinctly faster to their environment. Thus, the heating of the epidermis is lower than in the hair and its direct environment.

In one embodiment, a hair removal device can be configured to use intense light pulses having an energy density that does not necessitate a cooling of the skin surface during the treatment. A hair removal device having a radiation source configured to emit radiation having an energy density of between about 2 J/cm² and about 25 J/cm² can be used to reduce hair growth of a subject. In many instances, this device can be used to treat the skin of the user without requiring the use of a coolant as is used with many commercially available products. Referring again to Fig. 1, the light source 12 can be a light source emitting light having an energy density of between about 2 J/cm² and about 25 J/cm², for example the light source can have an energy density of between about 5 J/cm² and about 20 J/cm², between about 8 J/cm² and about 22 J/cm², or between about 12 J/cm² and about 18 J/cm².

In some embodiments, the light source has a beam area of less than about 1 mm², for example, less than about 0.9 mm², less than about 0.8 mm², less than about 0.7 mm², less than about 0.6 mm², less than about 0.5 mm², less than about 0.4 mm², less than about 0.3 mm², etc. In instances where energy densities of between about 2 J/cm² and about 25 J/cm² are used, light efficiencies of only a few W are required, for example less than about 5W, less than about 4W, less than about 3W, less than about 2W, less than about 1 W, or less than about 0.5W. Thus, the heat can be supplied to the skin per light pulse in a smaller quantity than with some other commercially available devices. Additionally, the supplied heat can flow off much more quickly due to a smaller beam area, which can allow the treatment to cause less pain than with some commercially available methods. In some instances, the methods can be substantially free of pain. In some embodiments, thermal damaging of the epidermis is also avoided.

In order to be able to treat skin areas larger than 1 mm², the light beam can be moved with a scanner over an area of some cm². For example, device can be used to cover an area of skin that is great than about 1 mm², for example an area of skin greater than about 0.2 mm², about 0.3 mm², about 0.5 mm², about 1.0 mm², about 2.0 mm², about 5.0 mm², about 7.0 mm², about 10.0 mm², about 15.0 mm², about 20.0 mm², about 25.0 mm² or greater.

In general, each point of the scanned skin area can be treated with the light source for between about 10 ms and about 40 ms, for example between 15 ms and about 35 ms, between about 20 ms and 30 ms, or about 25 ms.

A number of embodiments of the invention have been described. Nevertheless, it will be understood that various modifications may be made without departing from the scope of the invention.

For example, a hair-removing devices in which the reduction of hair growth function and the shaving function are combined, in which case the user can select the desired mode of operation, for example by means of an adjustment member. Preferably, the energy density of the light source is also controllable by means of the control unit of such a hair-removing device, so that the energy density of the light source can be adapted to the desired mode of operation of the hair-removing device. If the hair-removing device has an epilation function or has been set as a reduction of hair growth of a device by the user, the hair removing device may also be provided, for example, with an automatic shaving function. If the hair-removing device has exclusively an epilation function, in which case exclusively the roots of the hairs are destroyed, the hairs will not disappear from the skin until after some time, so that the desired result is not achieved immediately. If the epilation function of the hair-removing device is automatically combined with a shaving function, it is not only the roots of the hairs which are destroyed, but the hairs are also burnt through adjacent the surface of the skin, so that the hairs are immediately removed from the skin and the desired result is achieved instantaneously.

## Claims

1. A device for reducing hair growth on a subject, the device comprising:
a housing (10) comprising an opening (16) to allow radiation (20) to pass therethrough;
a treatment light source (12) disposed within the housing (10);
a sensor light source disposed within the housing (10); and
an optical sensor,
**characterized in that**
said optical sensor is arranged for detecting the reflection and scattering properties of the subject at the treatment location from a reflected and scattered sensor beam emitted by the sensor light source, said optical sensor being conductively linked to the sensor light source and the treatment light source (12),
and wherein the treatment light source (12) and the sensor light source share at least a portion of an optical path disposed within the housing (10).

2. The device according to claim 1, wherein the sensor light source has a wavelength of between about 400 nm and about 800 nm.

3. The device according to claim 1 or claim 2, wherein the device is arranged to calculate the quotient of the scattered and reflected light.

4. The device according to any one of claims 1 - 3, wherein the optical sensor comprises a dual PIN diode with a sensitive centre area and a second circular sensitive area.

5. The device of any one of claims 1 to 4 further comprising a controller operably linked to the optical sensor and the treatment light source (12).

6. The device of claim 5 wherein the controller is configured to switch on the treatment light source (12) upon detection of scattered light from the skin of the user.

7. The device of any one of claims 1 to 6 wherein the controller is configured to activate the treatment light source (12) only when skin is determined to be in the treatment area.

8. The device of claim 1 wherein the treatment light source (12) is a light emitting diode.

9. The device of claim 1 wherein the treatment light source (12) is a laser.

10. The device of claim 1 wherein the treatment light source (12) is a glow discharge lamp.

11. The device of claim 10 wherein the discharge lamp is a pulsed glow lamp.

12. The device of claim 10 wherein the discharge lamp is a continuous glow lamp.

13. The device of claim 1 further comprising a motion sensor conductively linked to the optical sensor.

14. The device of claim 13 wherein the motion sensor is an optical or a mechanical sensor.

15. The device of claim 13 wherein the motion sensor is disposed within the housing (10) or on an outer surface of the housing (10).

## Patentansprüche

1. Vorrichtung zum Verringern des Haarwachstums eines Individuums, wobei die Vorrichtung umfasst:
ein Gehäuse (10), das eine Öffnung (16) aufweist, um eine Strahlung (20) durchzulassen;
eine Behandlungslichtquelle (12), die in dem Gehäuse (10) angeordnet ist;
eine Sensorlichtquelle, die in dem Gehäuse (10) angeordnet ist; und
einen optischen Sensor,
**dadurch gekennzeichnet, dass**
der optische Sensor dafür ausgelegt ist, die Reflexions- und Streueigenschaften des Individuums an der Behandlungsstelle von einem reflektierten und gestreuten Sensorstrahl, der von der Sensorlichtquelle emittiert wird, zu erfassen, wobei der optische Sensor leitend mit der Sensorlichtquelle und der Behandlungslichtquelle (12) verbunden ist,
und wobei die Behandlungslichtquelle (12) und die Sensorlichtquelle zumindest einen Abschnitt eines optischen Weges, der in dem Gehäuse (10) angeordnet ist, gemeinsam haben.

2. Vorrichtung nach Anspruch 1, wobei die Sensorlichtquelle eine Wellenlänge zwischen etwa 400 nm und etwa 800 nm aufweist.

3. Vorrichtung nach Anspruch 1 oder Anspruch 2, wobei die Vorrichtung so ausgerichtet ist, dass sie den Quotienten des gestreuten und reflektierten Lichts berechnet.

4. Vorrichtung nach einem der Ansprüche 1-3, wobei der optische Sensor eine duale PIN-Diode mit einem empfindlichen mittleren Bereich und einem zweiten kreisförmigen empfindlichen Bereich umfasst.

5. Vorrichtung nach einem der Ansprüche 1 bis 4, ferner eine Steuereinrichtung umfassend, die betriebsmäßig mit dem optischen Sensor und der Behandlungslichtquelle (12) verbunden ist.

6. Vorrichtung nach Anspruch 5, wobei die Steuereinrichtung konfiguriert ist, um die Behandlungslichtquelle (12) bei Erfassung von gestreutem Licht von der Haut des Nutzers einzuschalten.

7. Vorrichtung nach einem der Ansprüche 1 bis 6, wobei die Steuereinrichtung konfiguriert ist, um die Behandlungslichtquelle (12) nur dann zu aktivieren, wenn bestimmt wird, dass die Haut im Behandlungsbereich liegt.

8. Vorrichtung nach Anspruch 1, wobei die Behandlungslichtquelle (12) eine lichtemittierende Diode ist.

9. Vorrichtung nach Anspruch 1, wobei die Behandlungslichtquelle (12) ein Laser ist.

10. Vorrichtung nach Anspruch 1, wobei die Behandlungslichtquelle (12) eine Glimmentladungslampe ist.

11. Vorrichtung nach Anspruch 10, wobei die Entladungslampe eine gepulste Glimmlampe ist.

12. Vorrichtung nach Anspruch 10, wobei die Entladungslampe eine kontinuierliche Glimmlampe ist.

13. Vorrichtung nach Anspruch 1, ferner einen Bewegungssensor umfassend, der leitend mit dem optischen Sensor verbunden ist.

14. Vorrichtung nach Anspruch 13, wobei der Bewegungssensor ein optischer oder mechanischer Sensor ist.

15. Vorrichtung nach Anspruch 13, wobei der Bewegungssensor im Gehäuse (10) oder an einer Außenfläche des Gehäuses (10) angeordnet ist.

## Revendications

1. Dispositif pour réduire la pousse des poils d'un sujet, le dispositif comprenant :
un logement (10) comprenant une ouverture (16) pour permettre à un rayonnement (20) de passer à travers ;
une source lumineuse de traitement (12) disposée au sein du logement (10) ;
une source lumineuse de capteur disposée au sein du logement (10) ; et
un capteur optique,
**caractérisé en ce que**
ledit capteur optique est arrangé pour détecter les propriétés de réflexion et de diffusion du sujet à l'emplacement de traitement à partir d'un faisceau de capteur réfléchi et diffusé émis par la source lumineuse de capteur, ledit capteur optique étant lié de manière conductrice à la source lumineuse de capteur et à la source lumineuse de traitement (12),
et dans lequel la source lumineuse de traitement (12) et la source lumineuse de capteur partagent au moins une partie d'une trajectoire optique disposée au sein du logement (10).

2. Dispositif selon la revendication 1, dans lequel la source lumineuse de capteur a une longueur d'onde comprise entre environ 400 nm et environ 800 nm.

3. Dispositif selon la revendication 1 ou la revendication 2, où le dispositif est arrangé pour calculer le quotient de la lumière diffusée et réfléchie.

4. Dispositif selon l'une quelconque des revendications 1 à 3, dans lequel le capteur optique comprend une diode PIN double avec une zone centrale sensible et une deuxième zone sensible circulaire.

5. Dispositif selon l'une quelconque des revendications 1 à 4, comprenant en outre un dispositif de commande lié de manière fonctionnelle au capteur optique et à la source lumineuse de traitement (12).

6. Dispositif selon la revendication 5, dans lequel le dispositif de commande est conçu pour activer la source lumineuse de traitement (12) lors d'une détection de lumière diffusée provenant de la peau de l'utilisateur.

7. Dispositif selon l'une quelconque des revendications 1 à 6, dans lequel le dispositif de commande est configuré pour activer la source lumineuse de traitement (12) uniquement lorsqu'on détermine que la peau est dans la zone de traitement.

8. Dispositif selon la revendication 1, dans lequel la source lumineuse de traitement (12) est une diode électroluminescente.

9. Dispositif selon la revendication 1, dans lequel la source lumineuse de traitement (12) est un laser.

10. Dispositif selon la revendication 1, dans lequel la source lumineuse de traitement (12) est une lampe à décharge luminescente.

11. Dispositif selon la revendication 10, dans lequel la lampe à décharge est une lampe à lueur pulsée.

12. Dispositif selon la revendication 10, dans lequel la lampe à décharge est une lampe à lueur continue.

13. Dispositif selon la revendication 1, comprenant en outre un capteur de mouvement lié de manière conductrice au capteur optique.

14. Dispositif selon la revendication 13, dans lequel le capteur de mouvement est un capteur optique ou mécanique.

15. Dispositif selon la revendication 13, dans lequel le capteur de mouvement est disposé au sein du logement (10) ou sur une surface externe du logement (10).
